# EUROPEAN PATENT SPECIFICATION

(11) **EP 3 366 233 B1**
(45) Date of publication and mention of the grant of the patent: **25.08.2021**
(21) Application number: 18158350.1
(22) Date of filing: 23.02.2018
(51) Int. Cl.: A61B 17/115, A61B 17/00

(54) **TOOL ASSEMBLY INCLUDING AXIALLY SPACED SPLINES**
WERKZEUGANORDNUNG MIT AXIAL BEABSTANDETEN KEILVERZAHNUNGEN
ENSEMBLE D'OUTIL COMPRENANT DES CANNELURES ESPACÉES AXIALEMENT

(30) Priority: 24.02.2017 US 201715441994
(43) Date of publication of application: 29.08.2018
(73) Proprietor: Covidien LP, Mansfield, MA 02048 (US)
(72) Inventor: GUERRERA, Joseph, Watertown, Connecticut 06795 (US); MOZDZIERZ, Patrick, Glastonbury, Connecticut 06033 (US)
(74) Representative: Soames, Candida Jane

(56) References cited:
- EP-A1- 2 813 187
- WO-A1-2014/139442
- CN-A- 104 905 837
- CN-U- 204 169 888
- US-A- 5 271 543

## Description

### BACKGROUND

### 1. Technical Field

The present disclosure relates to surgical stapling devices and, more specifically, to structures for aligning an anvil assembly with a cartridge assembly of a circular stapling device.

### 2. Discussion of Related Art

Circular stapling devices are employed by surgeons to apply one or more surgical fasteners, e.g., staples or two-part fasteners, to body tissue for the purpose of joining segments of body tissue together and/or for the creation of anastomoses. Circular stapling devices generally include an annular fastener cartridge assembly that supports a plurality of annular rows of fasteners, an annular anvil assembly operatively associated with the fastener cartridge assembly which provides a surface against which the fasteners are formed upon firing of the circular stapling device, and an annular blade for cutting tissue.

During a typical stapling procedure, the anvil assembly is separated from the stapling device to be positioned within one of the segments of body tissue. The anvil assembly is then attached to the stapling device and the stapling device is actuated to approximate the anvil assembly in relation to the cartridge assembly, to form the fasteners, and/or to severe tissue with the annular blade. It is important to align the anvil assembly with the cartridge assembly to ensure the fasteners are properly aligned with staple pockets of the anvil assembly to facilitate proper formation of the fasteners upon actuation or firing of the stapling device.

From CN204169888U a tool assembly is known, comprising a cartridge assembly, a shell housing the cartridge assembly and defining a passage and an anvil assembly including an anvil and a center rod extending from the anvil. The center rod defines a longitudinal rod axis and includes an alignment portion sized to pass through the passage, the alignment portion including a first spline and a second spline, each of the first and second splines extending in a direction parallel to the rod axis, the first spline having a first leading portion and the second spline having a second leading portion, the first leading portion positioned along the rod axis proximal of the second leading portion, wherein the first spline includes a first spline body extending from the first leading portion along the rod axis having a first length and the second spline includes a second spline body extending from the second leading portion along the rod axis having a second length less than the first length. J

A continuing need exists for structures and methods for aligning the anvil assembly with the cartridge assembly to promote proper formation of fasteners.

### SUMMARY

According to the invention, a tool assembly includes a cartridge assembly, a shell, and an anvil assembly. The shell houses the cartridge assembly and defines a passage. The anvil assembly includes an anvil and a center rod extending from the anvil. The center rod defines a longitudinal rod axis and includes an alignment portion that is sized to pass through the passage. The alignment portion includes first and second splines that each extend in a direction parallel to the rod axis. The first spline has a first leading portion and the second spline has a second leading portion. The first leading portion is positioned along the rod axis proximal of the second leading portion. The first spline includes a first spline body extending from the first leading portion along the rod axis having a first length and the second spline includes a second spline body extending from the second leading portion along the rod axis having a second length less than the first length; wherein the alignment portion has a plurality of faces defining a polygonal cross-section transverse to the rod axis; wherein the first spline is disposed on a first face of the plurality of faces, the first face having a first width less than a width of adjacent faces, and the second spline disposed on a second face of the plurality of faces, the second face having a second width less than a width of adjacent faces; and furthermore wherein the shell includes alignment splines extending along an inner wall of the shell defining the passage, and the first leading portion being configured to engage the alignment splines to clock the anvil assembly relative to the cartridge assembly as the center rod passes through the passage of the shell.

The shell includes alignment splines that extend along an inner wall of the shell defining the passage. The first leading portion may be configured to engage the alignment splines to clock the anvil assembly relative to the cartridge assembly as the center rod passes through the passage of the shell. Each alignment spline may define a channel with adjacent alignment splines. Each channel may be sized to receive the first and second splines to rotatably fix the anvil assembly relative to the shell.

In some aspects, the first leading portion includes first and second facets that form a wedge. The first leading portion may be configured to engage a respective one of the alignment splines to clock the anvil assembly relative to the shell. Each of the alignment splines may include a leading portion having first and second alignment facets forming a wedge. The leading portion of the alignment splines may be configured to be engaged by one of the first or second facets of the first leading portion to guide the first spline into a channel adjacent the alignment spline. Alternatively, the first leading portion may be bullet nose shaped and configured to engage the alignment splines to guide the first spline into a channel adjacent the alignment spline.

In certain aspects, the first spline includes a first trailing end and the second spline includes a second trailing end. The first and second trailing ends may axially aligned along the rod axis. The first spline may include a spline body that extends from the first leading portion along the rod axis to define a first length and the second spline may include a second spline body that extends from the second leading portion along the rod axis to define a second length that is less than the first length.

In particular aspects, the plurality of faces of the alignment portion may include nine faces. The first spline is disposed on a first face of the plurality of faces that has a first width that is less than a width of adjacent faces. The second spline is disposed on a second face of the plurality of faces having a second width that is less than a width of adjacent faces.

In some aspects, the center rod includes a plurality of second splines. The leading portion of each of the second splines may be positioned along the rod axis distal to the leading portion of the first spline. The leading portions of each of the second splines may be axially aligned with one another along the rod axis.

In particular aspects, the anvil is tiltable relative to the center rod.

In another aspect of the present disclosure, a circular stapling device includes a handle, an elongate body, and a tool assembly. The elongate body extends from the handle. The tool assembly is supported by the elongate body and includes a cartridge assembly, a shell, and an anvil assembly. The shell is secured to a distal portion of the elongate body and houses the cartridge assembly. The shell assembly includes an inner wall that defines a passage. The anvil assembly includes an anvil and a center rod extending from the anvil. The center rod defines a longitudinal rod axis and includes an alignment portion that is sized to pass through the passage. The alignment portion includes a first spline and a second spline that each extend in a direction parallel to the rod axis. The first spline has a first leading portion and the second spline has a second leading portion. The first leading portion is positioned along the rod axis proximal of the second leading portion.

In aspects, the elongate body includes an anvil retainer and the center rod includes fingers that extend from the alignment portion away from the anvil. The fingers may be configured to releasably receive the anvil retainer. The anvil retainer may be configured to draw the center rod through the passage of the shell to approximate the anvil with the cartridge assembly. The finger may be sized to pass through and rotate within the passage.

In another aspect of the present disclosure, a method of aligning an anvil assembly with a cartridge assembly of a circular stapling device includes drawing a center rod of the anvil assembly through a passage of a shell until a first spline disposed on the center rod engages an alignment portion of the shell, clocking the anvil assembly with the shell, and approximating the anvil assembly relative to the cartridge assembly. The shell houses the cartridge assembly. Clocking the anvil assembly with the shell includes continuing to draw the center rod through the passage such that the first spline cooperates with the alignment portion to rotate the anvil assembly about a longitudinal rod axis that is defined by the center rod. Approximating the anvil assembly relative to the cartridge assembly includes continuing to draw the center rod through the passage such that a second spline of the center rod that is disposed distal to the first spline cooperates with the alignment portion to rotatably secure the anvil assembly relative to the shell.

In aspects, clocking the anvil assembly with the shell includes engaging a first alignment spline of the alignment portion with the first spline such that the first alignment spline guides the first spline into a passage defined between the first alignment spline and a second alignment spline of the alignment portion. Guiding the first spline into the passage may include a leading portion of the first spline engaging a leading portion of the first alignment spline. The leading portion of the first spline may include first and second facets that form a wedge.

In another aspect of the present disclosure, a tool assembly includes a shell and an insert. The shell defines a longitudinal axis and includes an inner portion formed from a first material. The inner portion of the shell defines a lumen. The insert is secured within the lumen and has an inner surface that defines a passage. The inner surface includes a plurality of alignment splines. At least a portion of the insert is formed of a second material that is different from the first material.

In aspects, the insert has a proximal end and a distal end with each of the alignment splines extending a length of the insert between the proximal and distal ends. The alignment splines may extend distally from a distal end of the insert. Each of the alignment splines may extend in a direction parallel to the longitudinal axis.

In some embodiments, the second material has a hardness that is greater than a hardness of the first material. The second material may have a modulus of elasticity that is less than a modulus of elasticity of the first material. The second material may be a metal. The second material may be a thermoset plastic, surgical steel, stainless steel, or titanium. The first material may be a plastic. For example, the first material may be an injection molded plastic.

In particular aspects, the insert includes alignment splines that extend into the passage. The leading portion may be configured to engage the alignment splines to clock the anvil assembly relative to the cartridge assembly as the center rod passes through the passage of the insert. The leading portion may have a bullet nose shaped and be configured to engage a respective one of the alignment splines to clock the anvil assembly relative to the shell.

In another aspect of the present disclosure, a circular stapling device includes a handle, an elongate body, and a tool assembly. The elongate body extends from the handle and the tool assembly is supported by the elongate body. The tool assembly includes a shell and an insert. The shell defines a lumen about a longitudinal axis of the shell and is formed of a first material. The insert is secured within the lumen of the shell and has an inner surface that defines a passage about the longitudinal axis. The insert includes a plurality of alignment splines that protrude from the inner surface. At least a portion of the insert is formed of a second material that is different from the first material.

In another aspect of the present disclosure, a method of aligning an anvil assembly with a cartridge assembly of a circular stapling device includes drawing a center rod of the anvil assembly through a passage of a shell until a first spline disposed on the center rod engages an alignment mechanism of an insert received with the shell and clocking the anvil assembly with the shell by continuing to draw the center rod through the passage such that the first spline cooperates with the alignment mechanism to rotate the anvil assembly about a longitudinal rod axis defined by the center rod. The shell housing the cartridge assembly. The shell is formed of a first material and the insert is formed of a second material different from the first material.

In aspects, clocking the anvil assembly with the shell includes engaging a first alignment spline of the alignment mechanism with the first spline such that the first spline such that the first alignment spline guides the first spline into a passage defined between the first alignment spline and a second alignment spline of the alignment mechanism. Guiding the first spline into the passage may include a leading portion of the first spline engaging a leading portion of the first alignment spline.

Further, to the extent consistent, any of the aspects described herein may be used in conjunction with any or all of the other aspects described herein.

### BRIEF DESCRIPTION OF THE DRAWINGS

Various aspects of the present disclosure are described hereinbelow with reference to the drawings, which are incorporated in and constitute a part of this specification, wherein:
FIG. 1 is a perspective view of an exemplary circular stapling device including a tool assembly provided in accordance with the present disclosure;
FIG. 2 is an enlarged view of the indicated area of detail of FIG. 1 illustrating the tool assembly in a clamped configuration;
FIG. 3 is perspective view of the tool assembly with an anvil assembly separated from a shell;
FIG. 4 is an enlarged view of the indicated area of detail of FIG. 3;
FIG. 5 is a side view of the anvil assembly of FIG. 3;
FIG. 6 is an end, perspective view of the shell of FIG. 3 with a cartridge assembly removed;
FIG. 7 is a side view of the tool assembly of FIG. 3 with a center rod of the anvil assembly partially received within the shell;
FIG. 8 is a cross-sectional view taken along section line 8-8 of FIG. 7;
FIG. 9 is a perspective cut-away view of the tool assembly of FIG. 7 illustrating a dominant spline of the anvil assembly engaging alignment splines of the shell;
FIG. 10 is a perspective cut-away view of the tool assembly of FIG. 9 with the anvil assembly drawn into the shell with a body of the dominant spline positioned in a channel of the shell and a secondary spline of the anvil assembly engaging the alignment splines of the shell;
FIG. 11 is a side view of the anvil assembly of FIG. 3 with another embodiment of dominant and secondary splines in accordance with the present disclosure;
FIG. 12 is an enlarged view of the indicated area of detail of FIG. 11;
FIG. 13 is a perspective partial cutaway view of another shell with an insert provided in accordance with the present disclosure;
FIG. 14 is a rear perspective view of the insert of FIG. 13; and
FIG. 15 is a front perspective view of the insert of FIG. 13.

### DETAILED DESCRIPTION

This disclosure relates generally to a tool assembly having alignment features to promote proper formation of fasteners. The tool assembly includes a shell, a cartridge assembly, and anvil assembly. The cartridge assembly is releasably housed within the shell and the anvil assembly is separable from the shell. The anvil assembly includes a center rod having a dominant spline and one or more secondary splines. The shell defines a passage for receiving the center rod of the anvil assembly and includes alignment splines that are disposed within the passage. The dominant spline is positioned to engage the alignment splines of the shell to clock the anvil assembly relative to the shell such that the anvil assembly is aligned with the cartridge assembly to properly form fasteners upon actuation of the tool assembly. The secondary splines engage the alignment splines subsequent to the dominant spline engaging the alignment splines to rotatably fix the anvil assembly in relation to the shell. Receipt of the secondary splines between adjacent alignment splines may also finely clock the anvil assembly relative to the shell.

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closest to the clinician and the term "distal" refers to the portion of the device or component thereof that is farthest from the clinician. In addition, as used in this description, the term "clock" refers to rotationally aligning or orientating two components with one another.

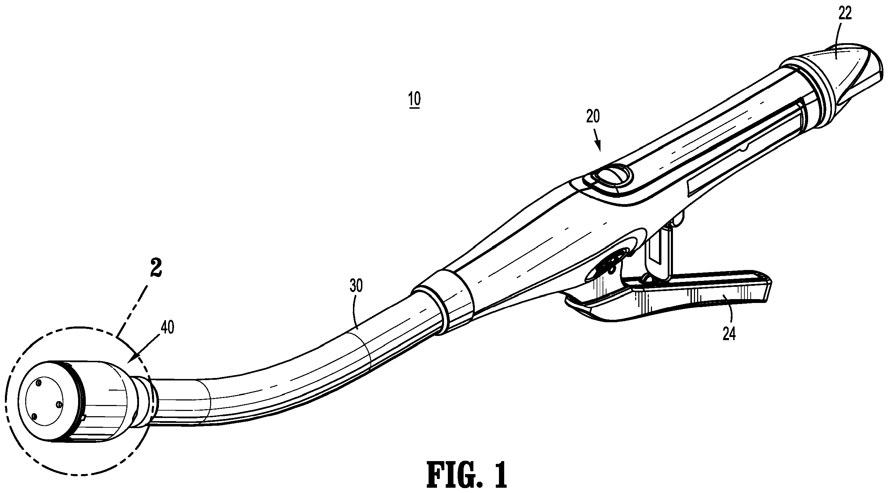

Referring initially to FIG. 1, a circular stapling device is disclosed herein and is generally designated as 10. In embodiments, the circular stapling device 10 is adapted for reuse and, in certain embodiments; the circular stapling device 10 is adapted for a single use and can be disposable.

The circular stapling device 10 includes a handle assembly 20, an elongate body 30, and a tool assembly 40. The tool assembly 40 can be provided as a removable and replaceable assembly that is secured to a distal portion of the elongate body 30. The handle assembly 20 includes a rotatable advancing member 22 and a pivotable trigger member 24 that are operatively coupled to drivers supported within the elongate body 30 to effectuate approximation of the tool assembly 40 and firing of the surgical stapling device 10. The elongate body 30 extends distally from a distal portion of the handle assembly 20 to a proximal portion of the tool assembly 40 so that the elongate body 30 is disposed between the handle assembly 20 and the tool assembly 40. In some embodiments, the elongate body 30 has a linear shape along the length of the elongate body 30, and in certain embodiments, the elongate body 30 has a curved shape along the length of the elongate body 30.

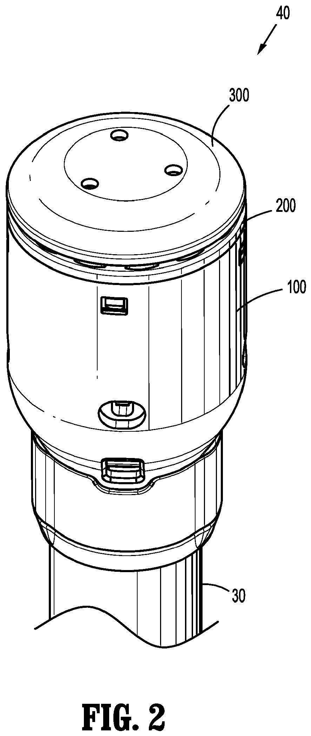

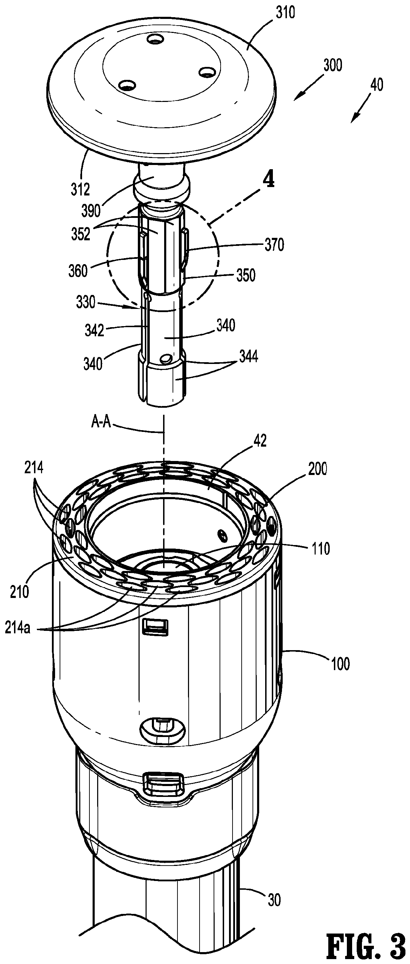

With reference to FIGS. 2 and 3, the tool assembly 40 includes a shell 100, a cartridge assembly 200, and an anvil assembly 300. In embodiments, the cartridge assembly 200 and/or the anvil assembly 300 may be replaced and the circular stapling device 10 may be reused. In some embodiment, the entire tool assembly 40 may be replaced such that the circular stapling device 10 may be reused. In embodiments, the tool assembly 40 includes a knife assembly with a substantially annular knife 42 adapted to cut tissue.

For a detailed discussion of the construction and operation of exemplary circular stapling devices reference may be made to U.S. Patent Nos. 5,915,616; 8,789,737 and 8,806,973.

The shell 100 houses the cartridge assembly 200 and receives a portion of the anvil assembly 300. The cartridge assembly 200 includes a tissue contacting surface 210 that defines a plurality of fastener retention slots 214. Each slot 214 defines an opening 214a. The openings 214a are arranged in coaxial annular rings positioned about the tissue contacting surface 210. As shown, the tissue contacting surface 210 defines three annular rings of openings 214a; however, it is contemplated that the tissue contacting surface 200 may include 1 or more annular rings of openings 214a, e.g., 2, 4, 5, etc. The cartridge assembly 200 further includes a fastener or a first part of a fastener (not shown) disposed within each of the retention slots 214.

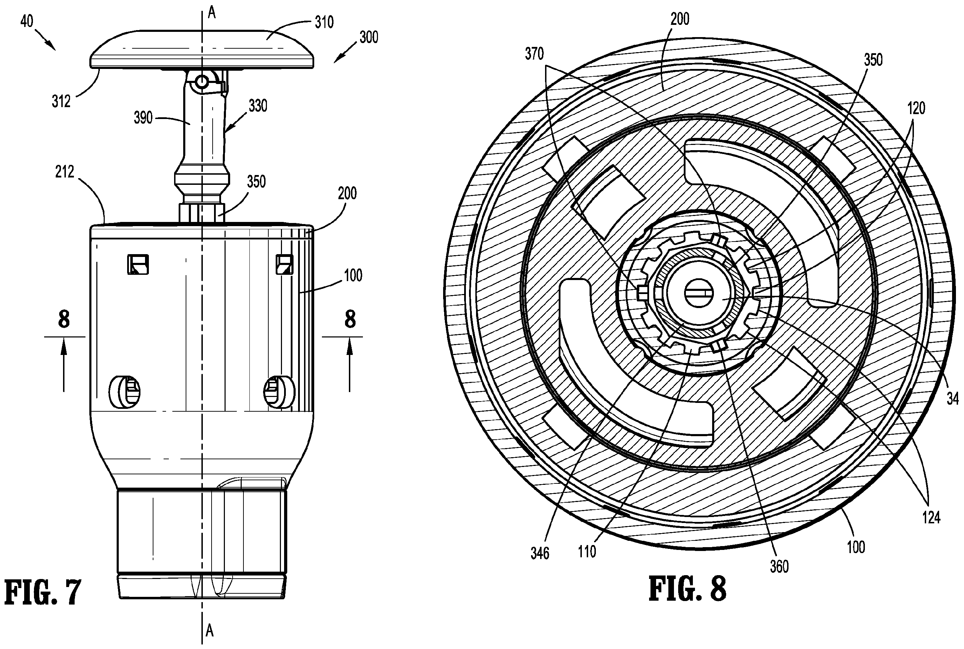

The anvil assembly 300 includes an anvil 310 and a center rod 330. The anvil 310 includes a tissue contacting surface 312 that defines a plurality of pockets (not shown) that align with the openings 214a of the cartridge assembly 200 to form fasteners upon actuation of the trigger 24 (FIG. 1). Alternatively, each of the pockets can include a second part of a fastener to form a completed fastener with a first part of a fastener disposed within one of the retention slots 214. The center rod 330 is pivotally coupled to the anvil 310 such that the anvil 310 can tilt relative to the center rod 330. Alternatively, the center rod 330 may be fixedly coupled to the anvil 310 such that the center rod 330 extends orthogonally away from the tissue contacting surface 312 of the anvil 310. The center rod 330 extends proximally from the anvil 310 and is configured to pass through the shell 100 and couple to an anvil retainer, e.g., a trocar 34 (FIG. 8), of the elongate body 30 (FIG. 1). The trocar 34 is configured to draw the anvil 310 to an approximated or clamped configuration (FIG. 2) in response to rotation of the rotatable advancing member 22 (FIG. 1).

The center rod 330 defines a longitudinal axis A-A (FIG. 3) of the anvil assembly 300 and includes fingers 340, an alignment portion 350, and a distal shaft 390. The alignment portion 350 is positioned between the distal shaft 390, which couples to the anvil 310, and the fingers 340. The fingers 340 define a cavity 346 (FIG. 8) that releasably receives the trocar 34. The fingers 340 each extend proximally from the alignment portion 350 to substantially form a cylinder with slits 342 defined between each of the fingers 340. The slits 342 permit the fingers 340 to expand outward to receive the trocar 34 within the cavity 346. A proximal portion of the fingers 340 includes a retention collar 344 which is configured to be received within the shell 100 when the trocar 34 is received within the fingers 34 to prevent the fingers 340 from expanding such that the trocar 34 can draw the anvil 310 to the clamped configuration adjacent the cartridge assembly 200 as shown in FIG. 2. The distal shaft 390 is substantially cylindrical in shape and is positioned along the longitudinal axis A-A.

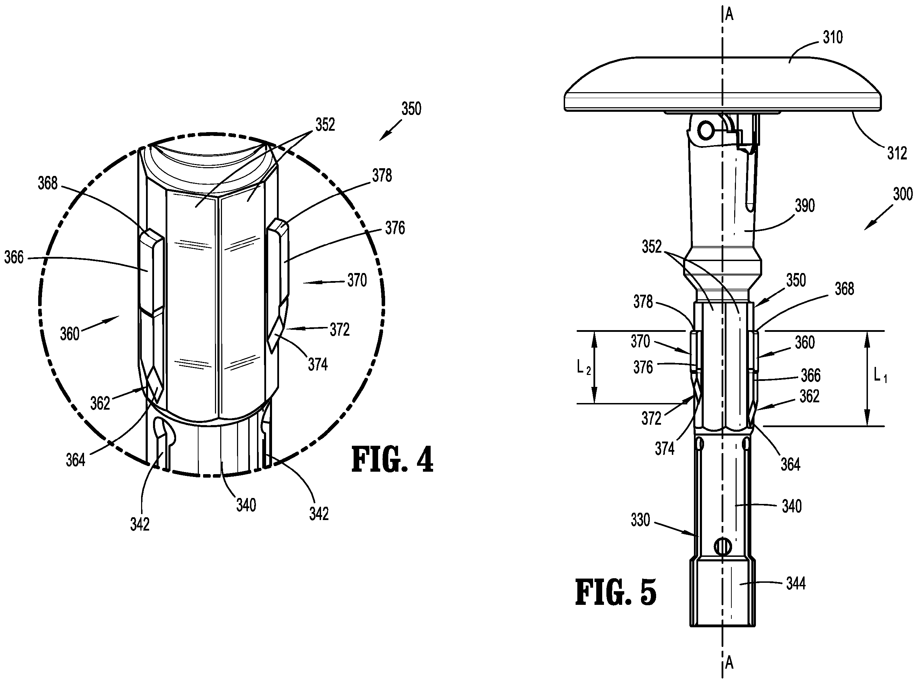

Referring to FIGS. 4 and 5, the alignment portion 350 of the anvil assembly 300 defines a plurality of faces 352 such that the alignment portion 350 has a polygonal cross-section. As shown, the alignment portion 350 has 9 faces 352; however, the alignment portion 350 can have a range of about 3 to about 18 faces. The alignment portion 350 includes a dominant spline 360 and secondary splines 370 which are each disposed on respective faces 352 of the plurality of faces 352 of the alignment portion 350 and are radially spaced apart from one another. As shown, the dominant and secondary splines 360, 370 are disposed on faces 352 having a width smaller than adjacent faces 352. This configuration may provide additional clearance for the alignment portion 350 within the shell 100 (FIG. 8) as detailed below. It is contemplated that each of the faces 352 of the alignment portion 350 may have an equal width. It is also contemplated that the alignment portion 350 of the anvil assembly 300 can be cylindrical and that the dominant spline 360 and the secondary splines 370 can be positioned along a cylindrical outer surface of the alignment portion 350.

The dominant spline 360 has a leading portion 362 including a pair of leading facets 364 forming a wedge and a body 366 extending distally from the leading facets 364 to a trailing end 368 of the body 366 in a direction parallel to the longitudinal axis A-A. The dominant spline 360 has a length L₁ defined along the longitudinal axis A-A from the leading facets 364 to the trailing end 368.

Each of the secondary splines 370 has a leading portion 372 including a pair of leading facets 374 forming a wedge and a body 376 extending distally from the leading facets 374 to a trailing end 378 of the body 376 in a direction parallel to the longitudinal axis A-A. The secondary splines 370 each have a length L₂ defined along the longitudinal axis A-A from the leading facets 374 to the trailing end 378 that is less than the length L₁. The leading facets 374 of the secondary splines 370 are positioned distal to the leading facets 364 of the dominant spline 360. The trailing ends 378 of the secondary splines 370 can be positioned along the longitudinal axis A-A at a position proximal, distal, or equal to the trailing end 368 of the dominant spline 360.

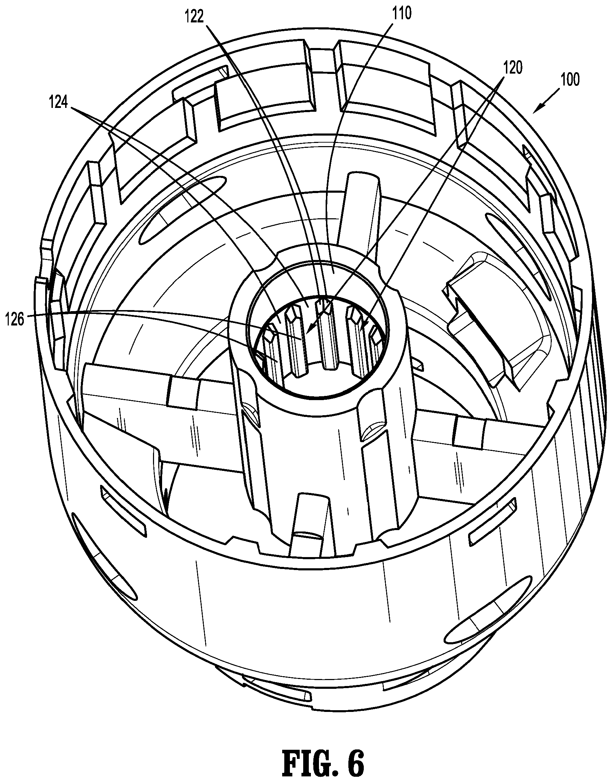

With reference to FIG. 6, the shell 100 defines a central passage 110 including a plurality of alignment splines 120 extending into the passage 110. The passage 110 is sized to permit the fingers 340 of the anvil assembly 300 to pass through the passage 110. Each of the alignment splines 120 is spaced apart from adjacent splines 120 to define channels 124 therebetween. Each alignment spline 120 includes a pair of alignment surfaces 122 that form a wedge and a body 126 extending proximally away from the alignment surfaces 122.

The channels 124 are sized and dimensioned to receive the body 366 of the dominant spline 360 and the bodies 376 of the secondary splines 370 to initially clock, i.e., align, the pockets of the anvil assembly 310 with the fastener retention slots 214 of the cartridge assembly 200 and, thereafter to fix the orientation the anvil assembly 300 relative to the shell 100 and the cartridge assembly 200. When the dominant and secondary splines 360, 370 are received within channels 124 of the shell 100, the fastener retention slots 214 (FIG. 3) of the cartridge assembly 200 are aligned with the pockets of the anvil 310 to promote proper fastener formation.

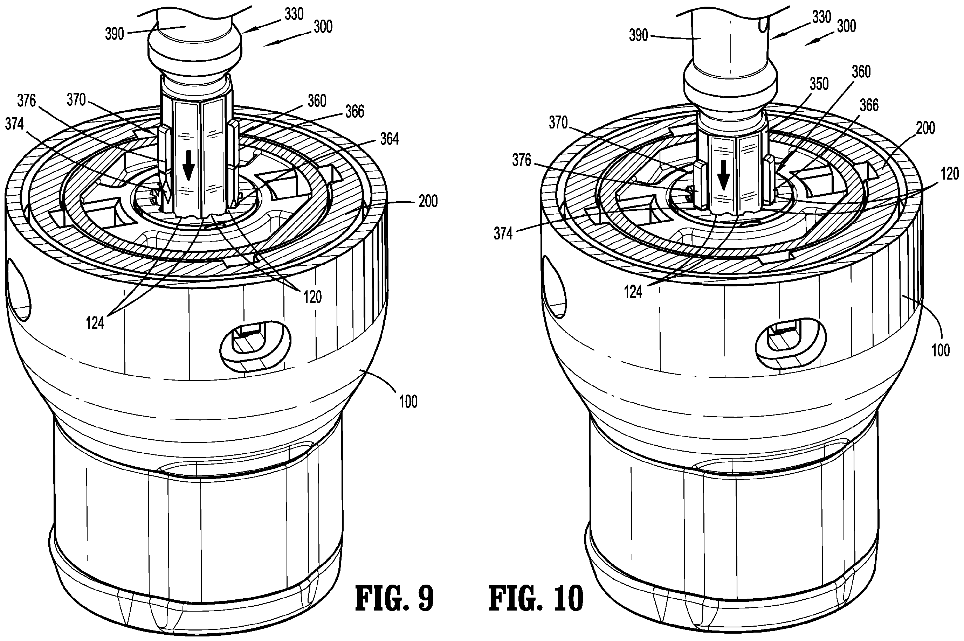

Referring to FIGS. 7-9, as the center rod 330 of the anvil assembly 300 is drawn through the passage 110 of the shell 100, the fingers 340 pass through the passage 110. After the fingers 340 pass beyond the alignment splines 120 within the passage 110, the leading facets 364 of the dominant spline 360 engage the alignment surfaces 122 of one or more of the alignment splines 120 if the body 366 is misaligned with the channels 124. Engagement between the leading facets 364 and the alignment surfaces 122 will effect rotation of the anvil assembly 300 about the center rod 330 to guide the body 366 of the dominant spline 360 into one of the channels 124 as shown in FIG. 9. As the leading facets 364 engage the alignment surfaces 122, the anvil assembly 300 rotates about the longitudinal axis A-A to clock the anvil assembly 300 relative to the shell 100 such that the body 366 of the dominant spline 360 and the bodies 376 of the secondary splines 370 are orientated to pass through the respective channels 124 (FIG. 8) between adjacent alignment splines 120. When the bodies 366, 376 are orientated to pass through respective channels 124, the pockets of the anvil 310 are aligned with the fastener retention slots 214 (FIG. 3) of the cartridge 200 such that fasteners will be properly formed when the fasteners are ejected or fired from the cartridge 200.

With particular reference to FIG. 9, the leading facets 364 of the dominant spline 360 are positioned proximal to the leading facets 374 of the secondary splines 370 such that the dominant spline 360 engages the alignment splines 120 before the secondary splines 370 to clock the anvil assembly 300 with the shell 100. By positioning the leading portion 362 of the dominant spline 360 at a position proximal of leading portions 372 of the the secondary splines 370, "crashing" of splines, caused by simultaneous engagement of multiple splines of the anvil assembly 310 with alignment splines 120 of the shell 100, is reduced or eliminated. "Crashing" of splines can increase resistance or, in some instances, result in binding of the center rod 330 within the shell 100 during retraction of the center rod 330 into the passage 110 of the shell 100. In addition, "crashing" of splines can increase particulate matter during a stapling procedure. If binding occurs, a clinician may be required to use additional force during retraction of the center rod 330 which may damage tissue, the shell 100, the cartridge assembly 200, or the anvil assembly 300.

Referring to FIG. 10, as the center rod 330 continues to be drawn through the passage 110 of the shell 100, the body 366 of the dominant spline 360 is received within a respective one of the channels 124 defined between adjacent alignment splines 120. Thereafter, the leading portion 372 of each of the secondary splines 370 enters a respective one of the channels 124 to align and rotatably fix the anvil assembly 300 with the shell 100. As the leading portions 372 of the secondary splines enter the respective channels 124, the orientation or clocking of the anvil assembly 300 relative to the shell 100 may be finely adjusted. When the center rod 330 is drawn through the passage 110 such that the anvil assembly 300 is in the clamped position (FIG. 2), the bodies 366, 376 are each disposed within a respective channel 124 of the shell 100 to align the pockets of the anvil assembly 330 with the fastener retention slots 214 (FIG. 3) of the cartridge assembly 200 and to rotationally fix the anvil assembly 330 relative to the cartridge assembly 200.

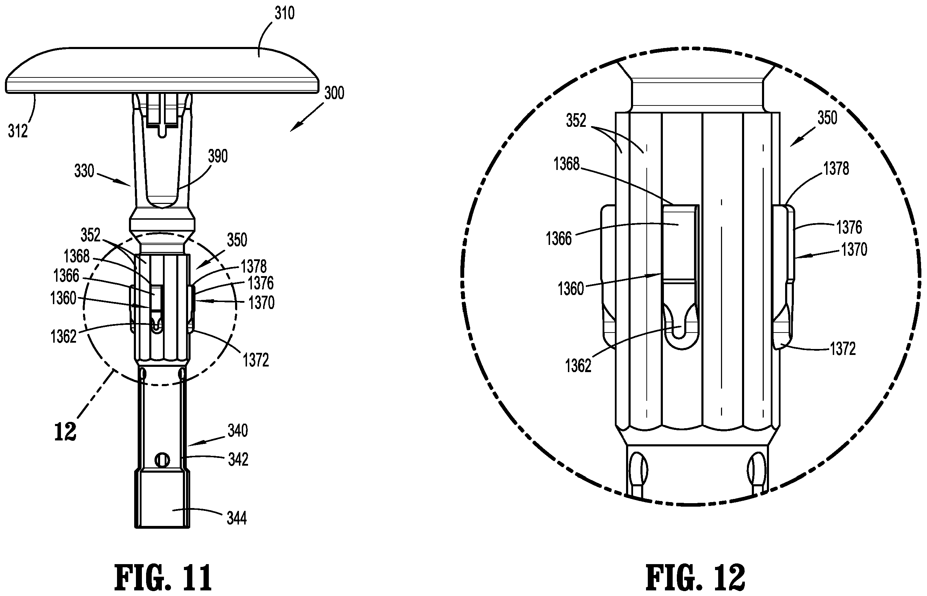

FIGS. 11 and 12 illustrate the anvil assembly 300 including another embodiment of a dominant spline 1360 and secondary splines 1370 in accordance with the present disclosure. The dominant and second splines 1360, 1370 are similar to the dominant and secondary splines 360, 370 detailed above with like structures represented with a similar label with an additional "1" preceding the previous label. Only the differences between splines 1360, 1370 and splines 360, 370 will be detailed below for brevity.

The dominant spline 1360 includes a leading portion 1362 and a trailing end 1368 with a body 1366 defined therebetween. The leading portion 1362 of the dominant spline 1360 has a bullet nose shape. The bullet nose shape of the leading portion 1362 is positioned on the alignment portion 350 to engage the alignment surfaces 122 of the alignment splines 120 of the shell 100 (FIG. 6) as the center rod 330 is drawn through the passage 110 of the shell 100 to clock or align the anvil assembly 300 relative to the shell 100. After the anvil assembly 300 and shell 100 are clocked, the body 1366 of the dominant spline 1360 is guided into a channel 124 defined between adjacent alignment splines 120 of the shell 100.

Each of the secondary splines 1370 includes a leading portion 1372 and a trailing end 1378 with a body 1376 defined therebetween. The leading portions 1372 of the secondary splines 1370 have a bullet nose shape. The bullet nose shape of the leading portions 1372 may engage the alignment surfaces 122 of the alignment splines 120 of the shell 100 (FIG. 6) as the center rod 330 is drawn through the passage 110 of the shell 100 to finely clock the anvil assembly 300 relative to the shell 100 such that the bodies 1376 of the secondary splines 1370 are each guided into a respective channel 124 of the shell 100.

The bullet nose shape of the leading portions 1362, 1372 is configured to reduce or minimize damage to the alignment splines 120 of the shell 100 caused by engagement of the dominant and secondary splines 1360, 1370 with the alignment splines 120 of the shell 100. In addition, the bullet nose shape of the leading portions 1362, 1372 may increase the detectability to a clinician of the "crashing" of splines by reducing an engagement force during clocking of the anvil assembly 300 and the shell 100 such that "crashing" of splines represents a distinct crashing force compared to an engagement force experienced during clocking. Further, the reduced engagement force during clocking may reduce particulate matter during a stapling procedure.

In some embodiments, the center rod 330 and the alignment splines 120 may be constructed of different materials. The bullet nose shape of the leading portions 1362, 1372 is configured to reduce or minimize damage in these embodiments. For example, a difference between the materials of the center rod 330 and the alignment splines 120 may allow edges of a leading portion of a dominant or secondary spline 360, 370 (e.g., an edge formed between leading facets 364 of dominant spline 360 (FIG. 4)) to deform or penetrate one or more alignment splines 120 causing damage to the alignment splines 120. Specifically, the shell 100 can be constructed of a material having a first hardness and a first modulus of elasticity and the center rod 330 can be constructed of a material having a second hardness and a second modulus of elasticity. The first hardness may be less than the second hardness and/or the first modulus of elasticity may be greater than the first modulus of elasticity. It is contemplated that the shell 100 may be formed from a plastic (e.g., an injection molded plastic) and the center rod 330 may be formed from a different plastic (e.g., a thermoset plastic) or a metal (e.g., surgical steel, stainless steel, or titanium).

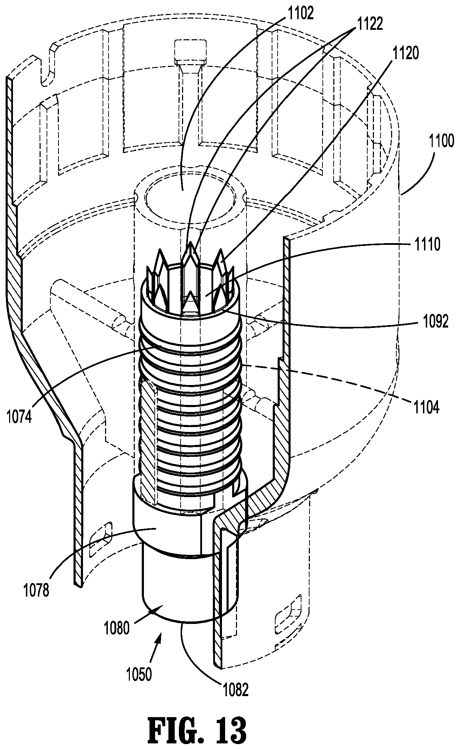

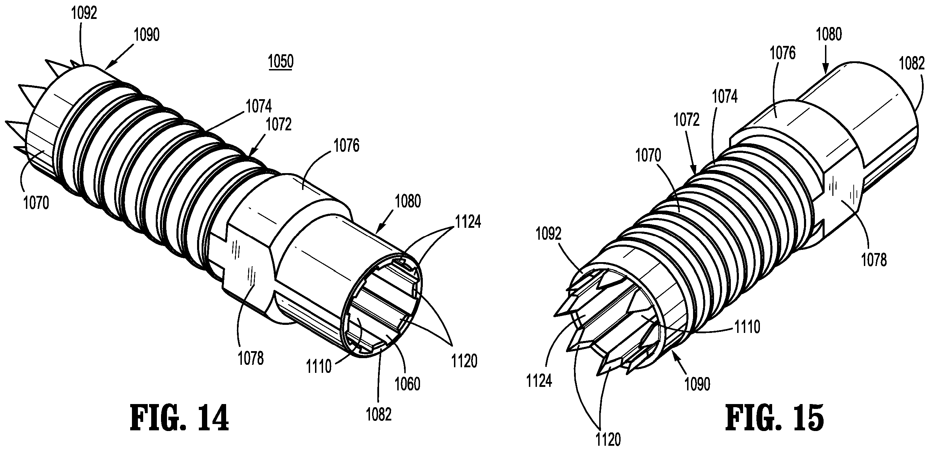

Referring to FIGS. 13-15 another shell 1100 is disclosed in accordance with the present disclosure. The shell 1100 is similar to the shell 100 detailed above with like elements labeled with an additional "1" before the previous label, as such, only the differences between shell 1100 and shell 100 will be detailed for reasons of brevity.

The shell 1100 defines a lumen 1102 that receives an insert 1050 and a longitudinal axis. The insert 1050 is substantially cylindrical in shape and has an inner surface 1060, an outer surface 1070, a proximal portion 1080, and a distal portion 1090. The inner surface 1060 defines a passage 1110 that extends along the longitudinal axis of the shell 1100. The insert 1050 includes alignment splines 1120 that protrude from the inner surface 1060 and in some embodiments extend in a direction parallel to the longitudinal axis along the entire length of the inner surface 1060 between the a proximal end 1082 and a distal end 1092 of the insert 1050 and may extend distally from the distal end 1092 of the insert 1050. The alignment splines 1120 are spaced about the inner surface 1060 to define channels 1124 between adjacent alignment splines 1120.

The outer surface 1070 of the insert 1050 includes retaining feature 1072 that secures the insert 1050 within the lumen 1102. As shown the retaining feature 1072 is in the form of a helical rib 1074 that is disposed about the outer surface 1070 of the insert 1050 such that the insert 1050 can be rotated into the lumen 1102 with the rib 1074 engaging a surface defining the lumen 1102 to secure the insert 1050 within the lumen 1102. The outer surface 1070 of the insert 1050 may include a stop 1076 in the proximal portion 1080 that abuts a portion of the shell 1100 when the insert 1050 is fully disposed within the lumen 1102. In some embodiments, the stop 1076 includes flats 1078 that can be engaged by a tool (not shown) to rotate the insert 1050 into the lumen 1102. In certain embodiments, the surface defining the lumen 1102 also defines a helical groove 1104 that receives the rib 1074 as the insert 1050 is rotated into the lumen 1102.

Additionally or alternatively, the rib 1074 may be formed of a first material and the shell 1100 may be formed of a second material different from the first material such that the rib 1074 bites into the surface defining the lumen 1102 as the insert 1050 is rotated into the lumen 1102. The first material may have a hardness greater than the second material and/or the first material may have a modulus of elasticity lower than the second material. For example, the first material may be a metal and the second material may be plastic.

It is contemplated that the alignment splines 1120 of the insert 1050 may also be formed from the first material. Forming the alignment splines 1120 of the first material may reduce or eliminate damage to the alignment splines 1120 experienced during clocking of the anvil assembly 300. Further, an insert 1050 formed of the first material may allow for higher clamping forces of tissue disposed between the anvil assembly 300 and the cartridge assembly 200 without experiencing deformation and/or damage to the shell 1100 when compared to the shell 100 detailed above.

It is contemplated that the entire shell 1100 may be formed of the second material, e.g., a plastic such as an injection molded plastic, and the rib 1074, the alignment splines 1120, and/or the entire insert 1050 may be formed of the first material, e.g., a thermoset plastic or a metal such as surgical steel, stainless steel, or titanium.

As described herein, the circular stapling device is a manually actuated stapling device; however it is contemplated that the shells 100, 1100 and/or the anvil assembly 300 can be used with a powered stapling device, such as an instrument with a motor, or being attachable to some power source. For a detailed description of an exemplary powered stapling device reference can be made to U.S. Patent Nos. 8,806,973 and 9,055,943.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned, the invention being limited by the scope of the appended claims. Therefore, the above description should not be construed as limiting, but merely as exemplifications of particular embodiments. Those skilled in the art will envision other modifications within the scope of the claims appended hereto.

## Claims

1. A tool assembly (40) comprising:
a cartridge assembly (200);
a shell (100) housing the cartridge assembly and defining a passage (110); and
an anvil assembly (300) including an anvil (310) and a center rod (330) extending from the anvil, the center rod defining a longitudinal rod axis (A-A) and including an alignment portion (350) sized to pass through the passage, the alignment portion including a first spline (360) and a second spline (370), each of the first and second splines extending in a direction parallel to the rod axis, the first spline having a first leading portion (362) and the second spline having a second leading portion (372), the first leading portion positioned along the rod axis proximal of the second leading portion,
wherein the first spline includes a first spline body (366) extending from the first leading portion (362) along the rod axis having a first length (L₁) and the second spline includes a second spline body (376) extending from the second leading portion along the rod axis having a second length (L₂) less than the first length ;
wherein the alignment portion (350) has a plurality of faces (352) defining a polygonal cross-section transverse to the rod axis (A-A); wherein the first spline (360) is disposed on a first face of the plurality of faces, the first face having a first width less than a width of adjacent faces, and the second spline (370) disposed on a second face of the plurality of faces, the second face having a second width less than a width of adjacent faces;
and furthermore wherein the shell (100) includes alignment splines (120) extending along an inner wall of the shell defining the passage (110), and the first leading portion (360) being configured to engage the alignment splines to clock the anvil assembly (300) relative to the cartridge assembly (200) as the center rod (330) passes through the passage of the shell.

2. The tool assembly (40) according to claim 1, wherein the alignment splines (120) each define a channel (124) with adjacent alignment splines, each of the channels being sized to receive the first and second splines (360, 370) to rotatably fix the anvil assembly (300) relative to the shell (100);

3. The tool assembly (40) according to claim 1 or claim 2 wherein the first leading portion (362) includes first and second facets (364) forming a wedge, the wedge being configured to engage a respective one of the alignment splines to clock the anvil assembly relative to the shell; preferably wherein each of the alignment splines includes a leading portion having first and second alignment facets forming a wedge, the wedge being configured to be engaged by a one of the first or second facets of the first leading portion to guide the first spline into a channel defined by adjacent ones of the alignment splines.

4. The tool assembly (40) according to claim 1 or claim 2, wherein the first leading portion (362) is bullet nose shaped and configured to engage a respective one of the alignment splines (120) to clock the anvil assembly (300) relative to the shell (100).

5. The tool assembly (40) according to any preceding claim, wherein the first spline (360) includes a first trailing end (368) and the second spline (370) includes a second trailing end (378), the first and second trailing ends being axially aligned along the rod axis (A-A).

6. The tool assembly (40) according to any preceding claim, wherein the center rod (330) includes a plurality of second splines (370), the leading portion (372) of each of the second splines positioned along the rod axis (A-A) at a position distal to the leading portion (362) of the first spline (360); preferably wherein the leading portion of each of the second splines is axially aligned with one another along the rod axis.

7. The tool assembly (40) according to any preceding claim, wherein the anvil (310) is tiltable relative to the center rod (330).

8. A circular stapling device (10) comprising:
a handle (20);
an elongate body (30) extending from the handle; and
a tool assembly (40) according to any preceding claim, said tool assembly supported by the elongate body.

9. The circular stapling device (10) according to claim 8, wherein the elongate body (30) includes an anvil retainer and the center rod (330) includes fingers (340) extending from the alignment portion away from the anvil (310), the fingers configured to releasably receive the anvil retainer, the anvil retainer configured to draw the center rod through the passage (110) of the shell (100) to approximate the anvil with the cartridge assembly (200); preferably wherein the fingers are sized to pass through and rotate within the passage.

## Patentansprüche

1. Werkzeuganordnung (40), die Folgendes umfasst:
eine Magazinanordnung (200);
eine Hülle (100), die die Magazinanordnung aufnimmt und einen Durchgang (110) definiert; und
eine Ambossanordnung (300), die einen Amboss (310) und einen Mittelstab (330) einschließt, der sich aus dem Amboss erstreckt, wobei der Mittelstab eine Längsstabachse (A-A) definiert und einen Ausrichtungsabschnitt (350) einschließt, der bemessen ist, um den Durchgang zu durchqueren, wobei der Ausrichtungsabschnitt einen ersten Keil (360) und einen zweiten Keil (370) einschließt, wobei sich der erste und der zweite Keil jeweils in eine Richtung parallel zu der Stabachse erstrecken, wobei der erste Keil einen ersten vorderen Abschnitt (362) aufweist und der zweite Keil einen zweiten vorderen Abschnitt (372) aufweist, wobei der erste vordere Abschnitt entlang der Stabachse proximal des zweiten vorderen Abschnitts positioniert ist,
wobei der erste Keil einen ersten Keilkörper (366) einschließt, der sich aus dem ersten vorderen Abschnitt (362) entlang der Stabachse erstreckt, der eine erste Länge (L₁) aufweist, und der zweite Keil einen zweiten Keilkörper (376) einschließt, der sich aus dem zweiten vorderen Abschnitt entlang der Stabachse erstreckt, der eine zweite Länge (L₂) aufweist, die geringer als die erste Länge ist;
wobei der Ausrichtungsabschnitt (350) mehrere Flächen (352) aufweist, die einen polygonalen Querschnitt quer zu der Stabachse (A-A) definieren; wobei der erste Keil (360) auf einer ersten Fläche der mehreren Flächen eingerichtet ist, wobei die erste Fläche eine erste Breite aufweist, die geringer als eine Breite angrenzender Flächen ist, und der zweite Keil (370) auf einer zweiten Fläche der mehreren Flächen eingerichtet ist, wobei die zweite Fläche eine zweite Breite aufweist, die geringer als eine Breite angrenzender Flächen ist;
und ferner wobei die Hülle (100) Ausrichtungskeile (120) einschließt, die sich entlang einer Innenwand der Hülle erstrecken, die den Durchgang (110) definiert, und wobei der erste vordere Abschnitt (360) konfiguriert ist, um die Ausrichtungskeile in Eingriff zu nehmen, um die Ambossanordnung (300) relativ zu der Magazinanordnung (200) zu takten, wenn der Mittelstab (330) den Durchgang der Hülle durchquert.

2. Werkzeuganordnung (40) nach Anspruch 1, wobei die Ausrichtungskeile (120) jeweils einen Kanal (124) mit angrenzenden Ausrichtungskeilen definieren, wobei jeder der Kanäle bemessen ist, um den ersten und den zweiten Keil (360, 370) aufzunehmen, um die Ambossanordnung (300) relativ zu der Hülle (100) drehbar zu befestigen;

3. Werkzeuganordnung (40) nach Anspruch 1 oder 2, wobei der erste vordere Abschnitt (362) erste und zweite Facetten (364) einschließt, die einen Absperrkörper ausbilden, wobei der Absperrkörper konfiguriert ist, um einen jeweiligen der Ausrichtungskeile in Eingriff zu nehmen, um die Ambossanordnung relativ zu der Hülle zu takten; vorzugsweise wobei jeder der Ausrichtungskeile einen vorderen Abschnitt einschließt, der erste und zweite Ausrichtungsfacetten aufweist, die einen Absperrkörper ausbilden, wobei der Absperrkörper konfiguriert ist, um durch eine der ersten oder der zweiten Facetten des ersten vorderen Abschnitts in Eingriff genommen zu werden, um den ersten Keil in einen Kanal zu leiten, der durch angrenzende Ausrichtungskeile definiert ist.

4. Werkzeuganordnung (40) nach Anspruch 1 oder 2, wobei der erste vordere Abschnitt (362) projektilförmig und konfiguriert ist, um einen jeweiligen der Ausrichtungskeile (120) in Eingriff zu nehmen, um die Ambossanordnung (300) relativ zu der Hülle (100) zu takten.

5. Werkzeuganordnung (40) nach einem der vorhergehenden Ansprüche, wobei der erste Keil (360) ein erstes hinteres Ende (368) einschließt und der zweite Keil (370) ein zweites hinteres Ende (378) einschließt, wobei das erste und das zweite hintere Ende entlang der Stabachse (A-A) axial ausgerichtet sind.

6. Werkzeuganordnung (40) nach einem der vorhergehenden Ansprüche, wobei der Mittelstab (330) mehrere zweite Keile (370) einschließt, wobei der vordere Abschnitt (372) von jedem der zweiten Keile entlang der Stabachse (A-A) an einer Position distal zu dem vorderen Abschnitt (362) des ersten Keils (360) positioniert ist; vorzugsweise wobei der vordere Abschnitt von jedem der zweiten Keile entlang der Stabachse aneinander axial ausgerichtet ist.

7. Werkzeuganordnung (40) nach einem der vorhergehenden Ansprüche, wobei der Amboss (310) relativ zu dem Mittelstab (330) kippbar ist.

8. Kreisförmige Klammernahtvorrichtung (10), die Folgendes umfasst:
einen Griff (20);
einen länglichen Körper (30), der sich aus dem Griff erstreckt; und
eine Werkzeuganordnung (40) nach einem der vorhergehenden Ansprüche, wobei die Werkzeuganordnung durch den länglichen Körper getragen wird.

9. Kreisförmige Klammernahtvorrichtung (10) nach Anspruch 8, wobei der längliche Körper (30) einen Ambosshalter einschließt und der Mittelstab (330) Finger (340) einschließt, die sich aus dem Ausrichtungsabschnitt weg von dem Amboss (310) erstrecken, wobei die Finger konfiguriert sind, um den Ambosshalter lösbar aufzunehmen, wobei der Ambosshalter konfiguriert ist, um den Mittelstab durch den Durchgang (110) der Hülle (100) zu ziehen, um den Amboss mit der Magazinanordnung (200) anzugleichen; vorzugsweise wobei die Finger bemessen sind, um den Durchgang zu durchqueren und sich innerhalb dessen zu drehen.

## Revendications

1. Ensemble outil (40) comprenant :
un ensemble cartouche (200) ;
une coque (100) logeant l'ensemble cartouche et définissant un passage (110) ; et
un ensemble enclume (300) comportant une enclume (310) et une tige centrale (330) s'étendant depuis l'enclume, la tige centrale définissant un axe de tige longitudinal (A-A) et comportant une partie d'alignement (350) dimensionnée pour passer à travers le passage, la partie d'alignement comportant une première cannelure (360) et une seconde cannelure (370), chacune des première et seconde cannelures s'étendant dans une direction parallèle à l'axe de tige, la première cannelure ayant une première partie avant (362) et la seconde cannelure ayant une seconde partie avant (372), la première partie avant étant positionnée le long de l'axe de tige à proximité de la seconde partie avant,
la première cannelure comportant un premier corps de cannelure (366) s'étendant à partir de la première partie avant (362) le long de l'axe de tige ayant une première longueur (L₁) et la seconde cannelure comportant un second corps de cannelure (376) s'étendant à partir de la seconde partie avant le long de l'axe de tige ayant une seconde longueur (L₂) inférieure à la première longueur ;
la partie d'alignement (350) ayant une pluralité de faces (352) définissant une section polygonale transversale à l'axe de tige (A-A) ; la première cannelure (360) étant disposée sur une première face de la pluralité de faces, la première face ayant une première largeur inférieure à une largeur de faces adjacentes, et la seconde cannelure (370) disposée sur une seconde face de la pluralité de faces, la seconde face ayant une seconde largeur inférieure à une largeur de faces adjacentes ;
et en outre la coque (100) comportant des cannelures d'alignement (120) s'étendant le long d'une paroi intérieure de la coque définissant le passage (110), et la première partie avant (360) étant configurée pour venir en prise avec les cannelures d'alignement afin de cadencer l'ensemble enclume (300) par rapport à l'ensemble cartouche (200) lorsque la tige centrale (330) passe à travers le passage de la coque.

2. Ensemble outil (40) selon la revendication 1, dans lequel les cannelures d'alignement (120) définissent chacune un canal (124) avec des cannelures d'alignement adjacentes, chacun des canaux étant dimensionné pour recevoir les première et seconde cannelures (360, 370) pour fixer de manière rotative l'ensemble d'enclume (300) par rapport à la coque (100) ;

3. Ensemble outil (40) selon la revendication 1 ou la revendication 2, dans lequel la première partie avant (362) comporte des première et seconde facettes (364) formant un coin, le coin étant configuré pour venir en prise avec une cannelure respective des cannelures d'alignement pour cadencer l'ensemble enclume par rapport à la coque ; de préférence, dans lequel chacune des cannelures d'alignement comporte une partie avant ayant des première et seconde facettes d'alignement formant un coin, le coin étant configuré pour être mis en prise par l'une des première ou seconde facettes de la première partie avant pour guider la première cannelure dans un canal défini par les cannelures d'alignement adjacentes.

4. Ensemble outil (40) selon la revendication 1 ou 2, dans lequel la première partie avant (362) est en forme de projectile et configurée pour venir en prise avec une cannelure respective des cannelures d'alignement (120) pour cadencer l'ensemble enclume (300) par rapport à la coque (100).

5. Ensemble outil (40) selon l'une quelconque des revendications précédentes, dans lequel la première cannelure (360) comporte une première extrémité arrière (368) et la seconde cannelure (370) comporte une seconde extrémité arrière (378), les première et seconde extrémités arrière étant alignées axialement le long de l'axe de tige (A-A).

6. Ensemble outil (40) selon l'une quelconque des revendications précédentes, dans lequel la tige centrale (330) comporte une pluralité de secondes cannelures (370), la partie avant (372) de chacune des secondes cannelures étant positionnée le long de l'axe de tige (A-A) à une position distale par rapport à la partie avant (362) de la première cannelure (360) ; de préférence dans lequel la partie avant de chacune des secondes cannelures est alignée axialement par rapport aux autres le long de l'axe de tige.

7. Ensemble outil (40) selon l'une quelconque des revendications précédentes, dans lequel l'enclume (310) peut être inclinée par rapport à la tige centrale (330).

8. Dispositif d'agrafage circulaire (10) comprenant :
une poignée (20) ;
un corps allongé (30) s'étendant depuis la poignée ; et
un ensemble outil (40) selon l'une quelconque des revendications précédentes, ledit ensemble outil étant supporté par le corps allongé.

9. Dispositif d'agrafage circulaire (10) selon la revendication 8, dans lequel le corps allongé (30) comporte un dispositif de retenue d'enclume et la tige centrale (330) comporte des doigts (340) s'étendant depuis la partie d'alignement à l'écart de l'enclume (310), les doigts étant configurés pour recevoir de manière amovible le dispositif de retenue d'enclume, le dispositif de retenue d'enclume étant configuré pour tirer la tige centrale à travers le passage (110) de la coque (100) pour rapprocher l'enclume de l'ensemble cartouche (200) ; de préférence dans lequel les doigts sont dimensionnés pour passer à travers et tourner à l'intérieur du passage.
